# EUROPÄISCHE PATENTSCHRIFT

(11) **EP 1 216 034 B1**
(45) Veröffentlichungstag und Bekanntmachung des Hinweises auf die Patenterteilung: **26.05.2004**
(21) Anmeldenummer: 00958534.0
(22) Anmeldetag: 13.09.2000
(51) Int. Cl.: A61K 9/70, B32B 31/00

(54) **VERFAHREN UND VORRICHTUNG ZUM SPENDEN HAFTKLEBENDER LAMINATABSCHNITTE**
METHOD AND DEVICE FOR DISPENSING ADHESIVE LAMINATE SEGMENTS FROM A MOVABLE PRIMARY SUPPORT SHEET SITUATED ON A SECONDARY SUPPORT SHEET
PROCEDE ET DISPOSITIF DE DISTRIBUTION DE PORTIONS DE STRATIFIE A ADHESION DE CONTACT A PARTIR D'UNE FEUILLE SUPPORT PRIMAIRE MOBILE SUR UNE FEUILLE SUPPORT SECONDAIRE MOBILE

(30) Priorität: 28.09.1999 DE 19946384
(43) Veröffentlichungstag der Anmeldung: 26.06.2002
(73) Patentinhaber: LTS LOHMANN Therapie-Systeme AG, 56626 Andernach (DE)
(72) Erfinder: VON FALKENHAUSEN, Christian, D-53125 Bonn (DE); SCHUMANN, Klaus, D-56567 Neuwied (DE); STEINBORN, Peter, D-56566 Neuwied (DE); THEOBALD, Frank, D-53498 Bad Breisig (DE)
(74) Vertreter: Flaccus, Rolf-Dieter, Dr.
(86) Internationale Anmeldenummer: PCT/EP2000/008927
(87) Internationale Veröffentlichungsnummer: WO 2001/022946

(56) Entgegenhaltungen:
- WO-A-97/22315
- US-A- 3 006 502

## Beschreibung

Die Erfindung betrifft ein Verfahren zum Spenden haftklebender Laminate oder Laminatabschnitte von einer beweglichen primären auf eine bewegliche sekundäre Trägerbahn, wobei die Laminate bei Umlenkung der primären Bahn um eine Spenderkante abgelöst und auf die sekundäre Bahn gespendet werden.

Die Herstellung und Verwendung flächig haftklebender Laminatabschnitte ist bekannt. Bei diesen kann es sich z.B. um Etiketten, Aufkleber, transdermale Therapiesysteme (TTS) oder doppelseitiges Klebeband handeln. Die Haftklebefläche bzw. eine haftklebend ausgebildete Matrix dieser Laminatabschnitte ist üblicherweise durch eine sie zumindest teilweise überragende Trägerbahn abgedeckt. Fertigungstechnisch werden diese Laminatabschnitte dadurch erzeugt, daß eine Matrix und eine mit ihr verbundene Rückschicht mit Hilfe eines geeigneten Werkzeugs bis zur Trägerbahn eingestanzt und ein überstehendes Gitter aus Matrix und einer Rückschicht abgezogen und verworfen werden.

Bei dieser Art der Fertigung von dermalen oder transdermalen therapeutischen, haftklebenden Laminatabschnitten kann es beim Durchstanzen der Matrix und einer mit ihr verbundenen Rückschicht zur Beschädigung der Oberflächenbeschichtung der Trägerbahn kommen, wodurch bei längerer Lagerung Trennprobleme zwischen Matrix und Trägerschicht auftreten, insbesondere dann, wenn das Therapiesystem eine starke Neigung zu kaltem Fluß aufweist, wodurch die Matrix mit einer nicht abhäsiv behandelten Trägerschicht ungewollt eine haftklebende Verbindung eingehen kann.

Dieses Problem kann jedoch umgangen werden, indem nach Vereinzelung der Abschnitte des Laminats durch Ausstanzen aus einem Ursprungslaminat die so erhaltenen transdermalen Pflaster anschließend auf eine endgültige, sekundäre Trägerschicht übertragen werden. Ein entsprechendes Verfahren ist in WO 97/22315 beschrieben. Das Verfahren setzt voraus, daß ein zu übertragendes transdermales therapeutisches System sich von einer primären Trägerbahn beim Führen über eine Spenderkante ablösen läßt, und somit auf die endgültige, sekundäre Trägerbahn übertragen werden kann. Falls jedoch die Haftkraft der Matrix einen bestimmten Betrag überschreitet, ist dieses Verfahren nicht mehr anwendbar, weil sich die haftklebende Matrix dann nicht mehr von der primären Trägerbahn ablöst, sondern mit der primären Trägerbahn umgelenkt wird.

Bei bekannten Spendeverfahren, wie beispielsweise in WO 97/22315 beschrieben, wird die primäre Trägerbahn mit vereinzelten Laminatabschnitten, z.B. Pflastern, in Transportrichtung über eine Spenderkante geführt, wobei das Pflaster über seine gesamte Breite von der Trägerbahn abgeschält wird. Dabei verläuft der Abschälvorgang in Bahnrichtung. Bei sehr starker Haftung des Pflasters an der primären Trägerschicht kann hierdurch selbst bei einem sehr spitzen Spendewinkel das Pflaster, ohne sich von der primären Trägerbahn abzulösen, mit um die Kante gezogen werden, so daß ein Spendevorgang nicht möglich ist.

Ausgehend vom Stand der Technik liegt der Erfindung die Aufgabe zugrunde, ein Verfahren und eine Vorrichtung der im Oberbegriff von Anspruch 1 angegebenen Art aufzuzeigen, welche die genannten Schwierigkeiten und technischen Grenzen überwinden und problemlos geeignet sind, selbst stark haftklebende Laminatabschnitte von ihrer Trägerbahn beim Umlenken um eine Spenderkante zu lösen und sie auf eine bewegliche sekundäre Trägerbahn zu spenden.

Die Aufgabe wird bei einem Verfahren der im Oberbegriff von Anspruch 1 genannten Art mit der Erfindung dadurch gelöst, daß die primäre Bahn in wenigstens zwei Streifen getrennt oder mit entsprechenden Sollbruchlinien ausgestattet wird, die Streifen einzeln an der Spenderkante umgelenkt und die haftklebenden Laminate dabei von den Streifen abgelöst und auf die Sekundärbahn gespendet werden.

Eine wesentliche Ausgestaltung des Verfahrens nach der Erfindung sieht vor, daß die Spenderkante so ausgebildet ist, daß die mindestens zwei Streifen der ersten Trägerbahn an jeweils eigenen Abschnitten der Spenderkante umgelenkt werden. Im einfachsten Fall besitzt die Spenderkante also zwei Abschnitte, an denen jeweils ein Streifen - vorzugsweise in unterschiedliche Richtung - umgelenkt wird. Dazu sind die beiden Abschnitte der Spenderkante nicht-linear angeordnet. zwischen den beiden Abschnitten der Spenderkante liegt ein Winkel, der zwischen 1° bis 179° oder 181° bis 359° liegt. Die Trennlinie bzw. die Sollbruchlinie zwischen beiden Streifen der ersten Trägerbahn verläuft in diesem Fall exakt über dem Schnittpunkt der beiden Abschnitte der Spenderkante.
Durch die Unterteilung der Primärbahn in wenigstens zwei Streifen, die einzeln von der Spenderkante abgezogen werden, ergibt sich eine Stabilisierung des Pflasters während des Spendevorgangs in Bahnrichtung. Dies wird bei nichtlinearer Ausbildung der Spenderkante dadurch begünstigt, daß die Streifen nicht in Bandlaufrichtung, sondern in je einem Winkel zur Bandlaufrichtung nach einer oder beiden Seiten abgezogen werden.

Eine alternative Ausgestaltung des Verfahrens, wobei die Primärbahn in wenigstens drei Streifen (d.h. mit mindestens zwei Trenn- bzw. Sollbruchlinien ausgestattet) getrennt wird, zeichnet sich dadurch aus, daß zuerst der oder die inneren Streifen der Primärbahn an einem ersten Abschnitt der Spenderkante umgelenkt und dabei von der Primärbahn und den darauf noch haftenden Laminaten abgezogen wird. Dabei bleiben die haftklebenden Laminate weiterhin mit den äußeren Streifen der Primärbahn verbunden. Die äußeren Streifen werden anschließend über weitere Abschnitte der Spenderkante umgelenkt, wobei die haftklebenden Laminate zuletzt abgelöst und auf die sekundäre Bahn gespendet werden.

Dadurch, daß jeder Streifen der primären Trägerbahn nur einen Teilbereich der Haftklebefläche des Laminatabschnittes überdeckt, der infolgedessen zum Ablösen nur einen Bruchteil der gesamten Haftkraft zu überwinden hat, kann das Ablösen eines stark haftklebenden Laminatabschnittes streifenweise simultan oder sukzessiv in mehreren Stufen ohne Schwierigkeiten durchgeführt werden.

Weil insbesondere bei einer sukzessiven Ablösung jeweils nur ein Bruchteil der Haftkraft überwunden werden muß, kann beim erfindungsgemäßen Verfahren auch von der Maßnahme Gebrauch gemacht sein, daß die primäre Trägerbahn relativ zur sekundären Trägerbahn mit einer geringeren Transportgeschwindigkeit geführt wird. Dies ermöglicht beispielsweise ein spätes Aufsetzen bzw. Anhaften eines haftklebenden Laminatabschnitts auf die sekundäre Trägerbahn, wobei nur noch ein geringer Teil auf der Primärbahn haftet. Hierdurch können weitgehende Unterschiede in den Bahngeschwindigkeiten zwischen Primär- und Sekundärbahn realisiert werden, wodurch eine Vergrößerung der Abstände zwischen den einzelnen Systemen ohne weitere Maßnahmen erreicht werden kann. Der Vorgang des Spendens kann im kontinuierlichen oder diskontinuierlichen Fertigungsbetrieb erfolgen.

Eine Vorrichtung zum Übertragen haftklebender Laminate oder Laminatabschnitte von einer beweglichen primären auf eine bewegliche sekundäre Trägerbahn, wobei die Laminate bei einer Umlenkung der primären Bahn um eine Spenderkante abgelöst und auf die sekundäre Bahn gespendet werden, umfassend eine primäre und eine sekundäre Trägerbahn und eine Spenderkante ist dadurch gekennzeichnet, daß sie wenigstens ein Trennmittel besitzt, das so angeordnet ist, daß die primäre Bahn bei ihrer Bewegung in Laufrichtung mit mindestens einer Trenn- und/oder einer Sollbruchlinie ausgestattet wird.

In einer weiteren Ausführungsform kann das Verfahren getaktet verlaufen, wobei die Spenderkante mit Hilfe eines eigenen Antriebs in einem ersten Takt in Bandlaufrichtung bewegt wird (im so genannten Vorlauf) und während oder nach dem Spenden der haftklebenden Laminate oder Laminatabschnitte in einem weiteren Takt entgegen der Bandlaufrichtung (im so genannten Rücklauf) in die Ausgangsposition zurück gebracht wird.

Weitere Ausgestaltungen der Vorrichtung sind in den Unteransprüchen angegeben.

## Patentansprüche

1. Verfahren zum Spenden haftklebender Laminate oder Laminatabschnitte von einer beweglichen primären auf eine bewegliche sekundäre Trägerbahn , wobei die Laminate bei Umlenkung der primären Bahn um eine Spenderkante abgelöst und auf die sekundäre Bahn gespendet werden, **dadurch gekennzeichnet, daß** die primäre Bahn mit mindestens einer Trenn- oder Sollbruchlinie ausgestattet und so in wenigstens zwei Streifen unterteilt wird und die Streifen einzeln von separaten Abschnitten der Spenderkante abgezogen werden.

2. Verfahren nach Anspruch 1, **dadurch gekennzeichnet, daß** die mindestens zwei Abschnitte der Spenderkante nicht-linear angeordnet sind.

3. Verfahren nach einem der Ansprüche 1 oder 2, **dadurch gekennzeichnet, daß** zwei Abschnitte der Spenderkante 4 einen Winkel zwischen 1° bis 179° oder 181° bis 359° aufspannen.

4. Verfahren nach einem der Ansprüche 1 bis 3, **dadurch gekennzeichnet, daß** zwei Abschnitte der Spenderkante in Bahnlaufrichtung einen Abstand aufweisen.

5. Verfahren nach einem oder mehreren der Ansprüche 1 bis 4, wobei die Primärbahn in wenigsten drei Streifen unterteilt wird, **dadurch gekennzeichnet, daß** zuerst der oder die inneren Streifen der Primärbahn (1) an einem ersten Abschnitt der Spenderkante umgelenkt wird, und daß die äußeren Streifen anschließend an weiteren Abschnitten der Spenderkante umgelenkt werden.

6. Verfahren nach einem oder mehreren der Ansprüche 1 bis 5, **dadurch gekennzeichnet, daß** die mindestens zwei Streifen der primären Bahn sukzessiv in mehreren Stufen von dem haftklebenden Laminat abgezogen werden.

7. Verfahren nach einem oder mehreren der Ansprüche 1 bis 6, **dadurch gekennzeichnet, daß** die mindestens zwei Streifen der primären Bahn simultan von dem haftklebenden Laminat abgezogen werden.

8. Verfahren nach einem oder mehreren der Ansprüche 1 bis 5, **dadurch gekennzeichnet, daß** die primäre Bahn relativ zur sekundären Trägerbahn mit der gleichen oder einer geringeren Transportgeschwindigkeit geführt wird.

9. Verfahren nach einem oder mehreren der Ansprüche 1 bis 8, **dadurch gekennzeichnet, daß** die primäre Bahn und/oder die sekundäre Bahn kontinuierlich oder diskontinuierlich transportiert werden.

10. Verfahren nach einem oder mehreren der Ansprüche 1 bis 9, **dadurch gekennzeichnet, daß** die Spenderkante in einem ersten Takt in Bandlaufrichtung bewegt wird und während oder nach dem Spenden der haftklebenden Laminate oder Laminatabschnitte in einem weiteren Takt entgegen der Bandlaufrichtung in die Ausgangsposition zurück gebracht wird.

11. Vorrichtung zum Übertragen haftklebender Laminate oder Laminatabschnitte von einer beweglichen primären auf eine bewegliche sekundäre Trägerbahn, wobei die Laminate bei einer Umlenkung der primären Bahn um eine Spenderkante abgelöst und auf die sekundäre Bahn gespendet werden, umfassend eine primäre und eine sekundäre Trägerbahn und eine Spenderkante , **dadurch gekennzeichnet, daß** sie ein Trennmittel besitzt, das so angeordnet ist, daß die primäre Bahn bei ihrer Bewegung in Laufrichtung mit mindestens einer Trenn- oder Sollbruchlinie ausgestattet wird.

12. Vorrichtung nach Anspruch 10, **dadurch gekennzeichnet, daß** das Trennmittel ein feststehendes Messer, eine rotierende Schneidewalze oder ein rotierendes Messer ist.

13. Vorrichtung nach Anspruch 10 oder 11, **dadurch gekennzeichnet, daß** die Spenderkante mindestens zwei nicht-linear angeordnete Abschnitte besitzt.

14. Vorrichtung nach einem oder mehreren der Ansprüche 10 bis 12, **dadurch gekennzeichnet, daß** die mindestens zwei Abschnitte einen Winkel zwischen 1° bis 179° oder 181° bis 359° aufspannen.

15. Vorrichtung nach einem oder mehreren der Ansprüche 10 bis 13, **dadurch gekennzeichnet, daß** die mindestens zwei Abschnitte in einem Abstand in Bahnlaufrichtung angeordnet sind.

16. Vorrichtung nach einem oder mehreren der Ansprüche 10 bis 14, **dadurch gekennzeichnet, daß** eine Trenn- oder Sollbruchlinie in der primären Bahn exakt über den Endpunkten der Abschnitte der Spenderkante verläuft.

17. Vorrichtung nach einem oder mehreren der Ansprüche 10 bis 15, **dadurch gekennzeichnet, daß** ein erster in einem mittleren Bereich angeordneter Abschnitt der Spenderkante und weitere im äußeren Bereich angeordnete Abschnitte der Spenderkante rechtwinklig zur Bandlaufrichtung ausgebildet sind.

18. Vorrichtung nach einem oder mehreren der Ansprüche 10 bis 16, **dadurch gekennzeichnet, daß** das V-förmige Profil einer Spenderkante beidseits von in Bandlaufrichtung verlaufenden Schnittlinien mindestens eine Stufe aufweist.

19. Vorrichtung nach einem oder mehreren der Ansprüche 10 bis 17, **dadurch gekennzeichnet, daß** die Spenderkante eine nach innen gerichtete Faltung in Bandlaufrichtung aufweist.

20. Vorrichtung nach einem oder mehreren der Ansprüche 10 bis 18, **dadurch gekennzeichnet, daß** die sekundäre Trägerbahn stromabwärts der Spenderkanten über eine Umlenkeinrichtung in Form einer Rolle oder einer abgerundeten Umlenkkante der Übertragungsstelle in Bandlaufrichtung zugeführt wird.

## Claims

1. Process for dispensing pressure-sensitive adhesive laminates or laminate sections from a movable primary onto a movable secondary carrier band, the laminates, upon deflection of the primary band around a dispenser edge, being detached and dispensed onto the secondary band, **characterized in that** the primary band is provided with at least one separation line or predetermined breaking line, thus subdividing it into at least two strips, and that the strips are individually pulled from separate sections of the dispenser edge.

2. Process according to Claim 1, **characterized in that** the at least two sections of the dispenser edge are arranged in a non-linear manner.

3. Process according to Claim 1 or 2, **characterized in that** two sections of the dispenser edge 4 span an angle of between 1° to 179° or 181° to 359°.

4. Process according to any one of Claims 1 to 3, **characterized in that** two sections of the dispenser edge are arranged at a distance from each other in the direction of travel of the band.

5. Process according to one or more of Claims 1 to 4, with the primary band being separated into at least three strips, **characterized in that** initially the inner strip or strips of the primary band is/are deflected at a first section of the dispenser edge, and that the outer strip is subsequently deflected at further sections of the dispenser edge.

6. Process according to one or more of Claims 1 to 5, **characterized in that** the at least two strips of the primary band are peeled from the pressure-sensitive adhesive laminate successively, in several stages.

7. Process according to one or more of Claims 1 to 6, **characterized in that** the at least two strips of the primary band are simultaneously peeled from the pressure-sensitive adhesive laminate.

8. Process according to one or more of Claims 1 to 5, **characterized in that** the primary band is directed, relative to the secondary carrier band, at a transport speed which is equal to or lower than that of the secondary carrier band.

9. Process according to one or more of Claims 1 to 8, **characterized in that** the primary band and/or the secondary band is/are conveyed continuously or intermittently.

10. Process according to one or more of Claims 1 to 9, **characterized in that** the dispenser edge is moved, in a first cycle, in the direction of travel of the band, and, during or after dispensing of the pressure-sensitive adhesive laminates or laminate sections, is returned, against the direction of travel of the band, to the start position in a further cycle.

11. Device for transferring pressure-sensitive adhesive laminates or laminate sections from a movable primary onto a movable secondary carrier band, the said laminates, upon deflection of the primary band around a dispenser edge, being detached and dispensed onto the secondary band, comprising a primary and a secondary carrier band and a dispenser edge, **characterized in that** it has a separation means which is arranged such that the primary band, during its movement in the direction of travel, is provided with at least one separation or predetermined breaking line.

12. Device according to Claim 10, **characterized in that** the separating means is a stationary knife, a rotating cutting roller or a rotating knife.

13. Device according to Claim 10 or 11, **characterized in that** the dispenser edge has at least two sections in non-linear arrangement.

14. Device according to one or more of Claims 10 to 12, **characterized in that** the at least two sections form an angle of between 1° to 179° or 181° to 359°.

15. Device according to one or more of Claims 10 to 13, **characterized in that** the at least two sections are arranged at a distance from each other, in the direction of travel of the band.

16. Device according to one or more of Claims 10 to 14, **characterized in that** a separation or predetermined breaking line runs in the primary band, exactly over the end points of the sections of the dispenser edge.

17. Device according to one or more of the Claims 10 to 15, **characterized in that** a first section of the dispenser edge, which section is arranged in a middle region, and further sections of the dispenser edge, which sections are arranged in the outer region, are configured at right angles to the direction of travel of the band.

18. Device according to one or more of Claims 10 to 16, **characterized in that** the V-shaped profile of a dispenser edge has at least one step on both sides of the cutting lines, which run in the direction of travel of the band.

19. Device according to one or more of Claims 10 to 17, **characterized in that** the dispenser edge has an inwardly facing fold in the direction of travel of the band.

20. Device according to one or more of Claims 10 to 18, **characterized in that**, downstream of the dispensing edges, the secondary carrier band is advanced in the direction of band travel to the transfer site via a deflecting device in the form of a roller or a rounded deflecting edge.

## Revendications

1. Procédé pour dispenser des stratifiés ou de sections de stratifié autoadhésifs, à partir d'une bande support primaire mobile sur une bande support secondaire mobile, les stratifiés étant détachés autour d'un bord dispensateur et dispensés sur la bande secondaire, lors d'un détournement de la bande primaire, **caractérisé en ce que** la bande primaire est munie d'au moins une ligne destinée à la séparation ou à la rupture et qu'elle est ainsi divisée en au moins deux rubans et **en ce que** les rubans sont retirés individuellement de sections séparées du bord dispensateur.

2. Procédé selon la revendication 1, **caractérisé en ce que** les sections du bord dispensateur qui sont au moins au nombre de deux ne sont pas disposées de façon linéaire.

3. Procédé selon l'une quelconque des revendications 1 ou 2, **caractérisé en ce que** deux sections du bord dispensateur 4 présentent un angle compris entre 1° et 179° ou 181° et 359 °.

4. Procédé selon l'une quelconque des revendications 1 à 3, **caractérisé en ce que** deux sections du bord dispensateur présentent un écart dans le sens de déplacement de la bande.

5. Procédé selon l'une quelconque ou plusieurs des revendications 1 à 4, la bande primaire étant divisée en au moins trois rubans, **caractérisé en ce que** dans un premier temps le ou les ruban(s) intérieurs de la bande primaire (1) est (sont) détournés dans une première section du bord dispensateur et **en ce que** les rubans extérieurs sont ensuite détournés dans d'autres sections du bord dispensateur.

6. Procédé selon l'une quelconque ou plusieurs des revendications 1 à 5, **caractérisé en ce que** les rubans de la bande primaire qui sont au moins au nombre de deux sont retirés successivement en plusieurs étapes du stratifié autoadhésif.

7. Procédé selon l'une quelconque ou plusieurs des revendications 1 à 6, **caractérisé en ce que** les rubans de la bande primaire qui sont au moins au nombre de deux sont retirés simultanément du stratifié autoadhésif.

8. Procédé selon l'une quelconque ou plusieurs des revendications 1 à 5, **caractérisé en ce que** la bande primaire est transportée à la même vitesse de transport ou à une vitesse de transport moindre, relativement à la bande support secondaire.

9. Procédé selon l'une quelconque ou plusieurs des revendications 1 à 8, **caractérisé en ce que** la bande primaire et/ou la bande secondaire sont transportées de façon continue ou discontinue.

10. Procédé selon l'une quelconque ou plusieurs des revendications 1 à 9, **caractérisé en ce que** lors d'un premier cycle, le bord dispensateur est déplacé dans le sens de déplacement de la bande et en cours de distribution des stratifiés ou des sections de stratifié autoadhésifs ou à l'issue de ladite distribution, au cours d'un autre cycle, il est ramené en sens contraire du sens de déplacement de la bande, dans la position initiale.

11. Dispositif pour le transfert de stratifiés ou de sections de stratifié autoadhésifs à partir d'une bande support primaire mobile sur une bande support secondaire mobile, lors d'un détournement de la bande primaire les stratifiés étant détachés autour d'un bord dispensateur et dispensés sur la bande secondaire, comprenant une bande support primaire et une bande support secondaire, **caractérisé en ce qu'**il est muni d'un agent de séparation, qui est disposé de façon à ce que lors de son déplacement dans le sens de déplacement, la bande primaire soit munie d'au moins une ligne destinée à la séparation ou à la rupture.

12. Dispositif selon la revendication 10, **caractérise en ce que** l'agent de séparation est une lame fixe, un cylindre de coupe rotatif ou une lame rotative.

13. Dispositif selon l'une quelconque des revendications 10 ou 11, **caractérisé en ce que** le bord dispensateur est muni d'au moins deux sections disposées de façon non linéaire.

14. Dispositif selon l'une quelconque ou plusieurs des revendications 10 à 12, **caractérisé en ce que** les sections qui sont au moins au nombre de deux présentent un angle compris entre 1 ° et 179 ° ou entre 181 ° et 359°.

15. Dispositif selon l'une quelconque ou plusieurs des revendications 10 à 13, **caractérisé en ce que** les sections qui sont au moins au nombre de deux sont disposées à distance dans le sens de déplacement de la bande.

16. Dispositif selon l'une quelconque ou plusieurs des revendications 10 à 14, **caractérisé en ce qu'**une ligne destinée à la séparation ou à la rupture dans la bande primaire s'étend exactement au-dessus des points d'extrémités des sections du bord dispensateur.

17. Dispositif selon l'une quelconque ou plusieurs des revendications 10 à 15, **caractérisé en ce qu'**une première section du bord dispensateur qui est disposée dans une zone centrale et d'autres sections du bord dispensateur qui sont disposées dans la zone externe sont conçues à la perpendiculaire du sens de déplacement de la bande.

18. Dispositif selon l'une quelconque ou plusieurs des revendications 10 à 16, **caractérisé en ce que** le profilé en V d'un bord dispensateur présente au moins un échelon, bilatéralement à des lignes de coupe qui s'étendent dans le sens de déplacement de la bande.

19. Dispositif selon l'une quelconque ou plusieurs des revendications 10 à 17, **caractérisé en ce que** le bord dispensateur est muni d'une convolution dirigée vers l'intérieur dans le sens de déplacement de la bande.

20. Dispositif selon l'une quelconque ou plusieurs des revendications 10 à 18, **caractérisé en ce qu'**en aval des bords dispensateur, la bande support secondaire est amenée dans le sens de déplacement de la bande par l'intermédiaire d'un dispositif de détournement sous forme d'un galet ou d'un bord de détournement arrondi de l'endroit de transfert.
